(19) Europäisches Patentamt
European Patent Office
Office européen des brevets

(11) Veröffentlichungsnummer: **0 046 185**
**A1**

(12) # EUROPÄISCHE PATENTANMELDUNG

(21) Anmeldenummer: **81105378.4**

(22) Anmeldetag: **10.07.81**

(51) Int. Cl.³: **C 07 C 85/145,** C 07 C 87/50, C 07 C 87/60, C 07 C 93/14

(30) Priorität: **18.08.80 DE 3031193**

(43) Veröffentlichungstag der Anmeldung: **24.02.82**
**Patentblatt 82/8**

(84) Benannte Vertragsstaaten: **CH DE FR GB IT LI**

(71) Anmelder: **BASF Aktiengesellschaft,**
**Carl-Bosch-Strasse 38, D-6700 Ludwigshafen (DE)**

(72) Erfinder: **Dimroth, Peter, Dr., Collinistrasse 5,**
**D-6800 Mannheim (DE)**
Erfinder: **Kemper, Reinhard, Dr., Viernheimer Weg 41,**
**D-6900 Heidelberg (DE)**
Erfinder: **Radtke, Volker, Dr., Barbarossastrasse 4,**
**D-6733 Hassloch (DE)**
Erfinder: **Puetter, Hermann, Dr., Landauer Strasse 59,**
**D-6730 Neustadt (DE)**

(54) **Verfahren zur Herstellung von substituierten 4,4'-Diaminodiphenylen.**

(57) Die Erfindung betrifft ein Verfahren zur Herstellung von Verbindungen der allgemeinen Formel I

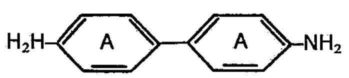

I,

in der die Ringe A mindestens einen Substituenten tragen, aus Verbindungen der Formel II

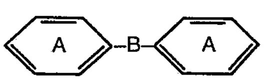

II,

in der

B einen Rest der Formeln

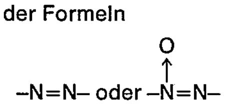

bedeutet;

das dadurch gekennzeichnet ist, daß man Verbindungen der Formel II in stark saurem Medium mit Titantrichlorid umsetzt.

EP 0 046 185 A1

# Verfahren zur Herstellung von substituierten 4,4'-Diaminodiphenylen

4,4'-Diaminodiphenyle sind wichtige Zwischenprodukte zur Herstellung von Farbstoffen und Pigmenten, sie werden in der Regel nach dem folgenden Reaktionsschema mit den Reaktionsschritten 1 und 2 hergestellt:

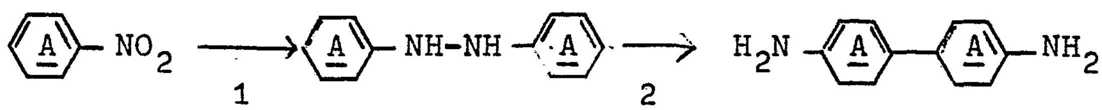


Schwierigkeiten treten vorwiegend beim Reaktionsschritt 1 auf.

Die Herstellung von z.B. 2,2'-Dichlorhydrazobenzol aus o-Nitrochlorbenzol gelingt mit Eisen in stark alkalischer Lösung (DRP 138 496). Bei der bekanntesten und in der Praxis am meisten ausgeübten Herstellungsweise wird mit Zinkstaub in Gegenwart von wäßriger Natronlauge, gegebenenfalls auch in Gegenwart eines Lösungsmittels, wie Solventnaphtha, reduziert.

Dieser Prozeß kann als Eintopf-Verfahren durchgeführt werden (BIOS 1153, S. 269 - 270; Winnacker-Küchler, Chem. Technologie, 1959, Bd. 3, S. 848 - 849; US-PS 2 640 081; Anilinokrasochnaya Prom. 4 (1934) 605 = C.A. 29 (1935), 2527; Poln. Pat. 53 687 (30.06.1965) = C.A. 68 (1968), 104 703 b). Schwierigkeiten liegen in einer guten Durchmischung der dickflüssigen Suspension und der Abführung der beträchtlichen Reaktionswärme, wodurch die Reaktion zum "Durchgehen" neigt.

Eine Verbesserung der Zinkstaub-Reduktion besteht darin, daß man die Reaktion kontinuierlich in einer vierstufigen Kaskade durchführt (DE-AS 2 140 785).

Die katalytische Direktreduktion von o-Nitrochlorbenzol zum 2,2-Dichlorhydrazobenzol gelingt nach US-PS 2 688 040 und US-PS 2 233 130 mit Palladium-Katalysatoren. Eine neuere Anmeldung (US-PS 3 156 724 vom 10.11.64) beschreibt die Reduktion in einem Zweiphasengemisch aus Toluol und wäßriger Natronlauge in Gegenwart eines Platin- oder Palladium-Katalysators und geringen Mengen an 2,3-Dichlor-1,4-naphthochinon.

Die Reduktion auf elektrochemischem Weg gelingt in alkalischer Lösung an Blei- oder Eisenkathoden (J. Sci & Ind. Research (India) 4 (1946), 645 = C.A. 40 (1946), 6347; Indian-PS 34 756 (28.07.1948) = C.A. 44 (1950), 6886. Vgl. auch Benzidinherstellung an rotierenden Kathoden: Chem.-Ing.-Techn. 41 (1969), 776).

Die Reduktion von z.B. 2,2'-Dichlorazoxybenzol zum 2,2'-Dichlorhydrazobenzol erreicht man außer mit Zink/Natronlauge oder Zink/Salzsäure auch mit Hydrazinhydrat/Raney-Nickel (DE-OS 2 609 530 mit Eisen/Säure (US-PS 3 919 701; C.A. 50 (1956), 9448), mit Aluminiumamalgam in Methanol (C.A. 50 (1956) 4223), mit technischem Natriumhydrosulfid BE-PS 832 269) oder einem Gemisch aus Natriumhydrosulfid und Natriumthiosulfat (BE-PS 832 268), mit Glukose/Natronlauge in Gegenwart von Kaliumcyanid und Dichlordicyanobenzochinon (US-PS 3 821 490), mit Dextrose/2,3-Dichlornaphthochinon in Methanol (US-PS 2 794 047) und mit Formaldehyd/2,3-Dichlornaphthochinon/Natronlauge in wäßrigem Alkohol (US-PS 2 794 046).

Die Erfindung betrifft nun ein Verfahren zur Herstellung von Verbindungen der allgemeinen Formel I

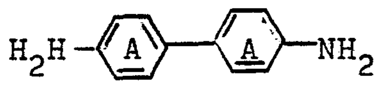

I,

in der die Ringe A mindestens einen Substiuenten tragen, aus Verbindungen der Formel II

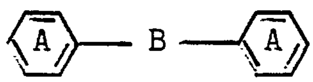

II,

in der
B einen Rest der Formeln

$$-N=N- \quad \text{oder} \quad -\overset{O}{\overset{\uparrow}{N}}=N- \quad \text{bedeutet,}$$

das dadurch gekennzeichnet ist, daß man Verbindungen der Formel II in stark saurem Medium mit Titantrichlorid umsetzt.
Für B ist Azoxy bevorzugt.
Die Ringe A können beispielsweise durch Fluor, Chlor, Brom, Alkyl, Alkoxy, Phenoxy oder Hydroxysulfonyl substituiert sein. Alkyl- und Alkoxyreste sind insbesondere solche mit 1 bis 4 C-Atomen, wie Butyl, Propyl, Ethyl, Butoxy, Propoxy und vorzugsweise Methyl, Methoxy oder Ethoxy.

Bevorzugt ist das erfindungsgemäße Verfahren zur Herstellung der Verbindungen der Formel I, die in 3,3'-Stellung durch Methyl, Methoxy und insbesondere Chlor substituiert sind.

Verbindungen der Formel II erhält man nach bekannten Methoden aus Nitrobenzolen der Formel

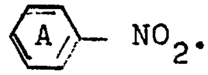

Als Reaktionsmedium für das erfindungsgemäße Verfahren eignen sich starke wäßrige anorganische Säuren, vorzugsweise Salzsäure, im Konzentrationsbereich von ungefähr 5 bis 40, vorzugsweise 12 bis 30 %.

Zweckmäßige Reaktionstemperaturen liegen im Bereich von Raum- bis zur Rückflußtemperatur, vorzugsweise wählt man Temperaturen zwischen 20 und 80°C.

Als besonders vorteilhaft hat es sich erwiesen, bei der erfindungsgemäßen Umsetzung ein Tensid zuzusetzen, es kommen dabei neutrale, anion- und kationaktive Verbindungen in Betracht.

Geeignete Tenside sind beispielsweise Ethoxylate von $C_9$-$C_{15}$-Oxoalkoholen, Fettalkoholethoxylate, Alkylphenolethoxylate, Dodecylbenzolsulfonsäure, Alkylnaphthalinsulfonsäuren sowie Tetraalkylammoniumsalze, die mindestens einen langkettigen Alkylrest enthalten.

Das Tensid wird zweckmäßigerweise in einer Menge von 0,5 bis 30 Gewichtsprozent, vorzugsweise 8 bis 20 Gewichtsprozent, bezogen auf die Menge der Verbindung der Formel II zugesetzt. Bevorzugt ist die Verwendung eines kationischen Tensids.

Zusätzlich kann die Reaktionslösung weiterhin wasserlösliche Lösungsmittel enthalten, wie Alkohole, Glykole, Di- oder Polyglykole, Dimethylformamid oder N-Methylpyrrolidon.

Schließlich können anstelle oder zusätzlich zu dem Lösungsmittel wasserlösliche Polymere enthalten sein, z.B. Polyvinylpyrrolidon oder Polyvinylimidazol.

Man erhält die Verbindungen der Formel I nach dem erfindungsgemäßen Verfahren in ausgezeichneten Ausbeuten von ungefähr

80 bis 95 %.
Das erfindungsgemäße Verfahren kann leicht kontinuierlich gestaltet werden, wenn man nach der Abtrennung der Verbindungen der Formel I die in der Reaktionslösung verbleibenden Titan-IV-salze elektrolytisch wieder zu Titan-III-salzen reduziert und die so reaktivierte Lösung wiederverwendet. Zweckmäßigerweise wird dabei jeweils ein geringer Teil des Reaktionsmediums entfernt und durch neues Gemisch ersetzt, um eine Konzentrierung der entstehenden Nebenprodukte zu vermeiden.

In den folgenden Beispielen beziehen sich Angaben über Teile und Prozente, sofern nicht anders vermerkt, auf das Gewicht.

Beispiel 1

Zu 240 Teilen einer 15 %igen $TiCl_3$-Lösung in 10 %iger Salzsäure werden 47 Teile 36 %ige Salzsäure, 2 Teile Dodecylbenzolsulfonsäure sowie 13,4 Teile 2,2'-Dichlorazoxybenzol gegeben. Nach 6stündigem Rühren bei 60°C ist die violette Lösung entfärbt. Durch Filtration und nach dem Waschen mit 5 %iger Salzsäure erhält man 13,7 Teile 3,3'-Dichlorbenzidindihydrochlorid, welches frei von o-Chloranilin ist.

Beispiel 2

Zu 225 Teilen einer 15 %igen $TiCl_3$-Lösung in 10 %iger Salzsäure werden 47 Teile 36 %ige Salzsäure, 2 Teile Dodecylbenzolsulfonsäure sowie 12,9 Teile 2,2'-Dimethoxyazoxybenzol gegeben. Nach 4stündigem Rühren bei 65°C ist die violette Lösung entfärbt. Nach dem Abfiltrieren und Waschen mit 5 %iger Salzsäure erhält man 12,4 Teile 3,3'-Dimethoxybenzidindihydrochlorid.

Beispiel 3

Zu 225 Teilen einer 15 %igen $TiCl_3$-Lösung in 10 %iger Salzsäure werden 47 Teile 36 %ige Salzsäure, 2 Teile Dodecylbenzolsulfonsäure sowie 11,3 Teile 2,2'-Dimethylazoxybenzol gegeben und dann 5 Stunden bei 70°C gerührt. Nach der Filtration und dem Waschen mit 5 %iger Salzsäure erhält man 10,8 Teile 3,3'-Dimethylbenzidindihydrochlorid.

Beispiel 4

In 1400 Teile einer 15 %igen $TiCl_3$-Lösung wird gasförmiger Chlorwasserstoff eingeleitet, bis die HCl-Konzentration 25 % entspricht. Nach Zugabe von 3 Teilen mit 8 Mol Ethylenoxid ethoxyliertem Nonylphenol als nichtionogenem Tensid wird mit 78 Teilen 2,2'-Dichlorazoxybenzol versetzt und 1 Stunde bei 60 - 65°C gerührt. Das ausgefallene 3,3'-Dichlorbenzidindihydrochlorid wird abfiltriert und mit 3 %iger Salzsäure gewaschen. Das feuchte Filtergut enthält 76,8 Teile reines 3,3'-Dichlorbenzidindihydrochlorid.

Beispiel 5

492 Teile einer 15 %igen $TiCl_3$-Lösung in 10 %iger Salzsäure werden mit 114 Teilen 36 %iger Salzsäure, 45 Teilen Diethylenglykol sowie 4 Teilen Dimethyl-$C_{12}$-$C_{14}$-alkyl-benzylammoniumchlorid als kationischem Tensid versetzt. Nach Zugabe von 26,8 Teilen 2,2'-Dichlorazoxybenzol wird 4 Stunden bei 60°C gerührt. Es fallen 27,6 Teile 3,3'-Dichlorbenzidindihydrochlorid aus, die durch Absaugen isoliert und mit 5 %iger Salzsäure gewaschen werden.

Beispiel 6

1440 Teile einer 20 %igen Salzsäurelösung, die 144 Teile Titantrichlorid gelöst enthält, wird mit 5 Teilen Dimethyl-$-C_{12}-C_{14}$-alkyl-benzyl-ammoniumchlrid sowie 50 Teilen 2,2'-Dichlorazoxybenzol versetzt und 2 Stunden bei 55 - 60°C gerührt. Nach dem Abfiltrieren und Waschen mit 5 %iger Salzsäure enthält das feuchte Filtergut 51 Teile 3,3'-Dichlorbenzidindihydrochlorid.

Beispiel 7

Die Mutterlauge der Filtration aus Beispiel 6 wird in eine Elektrolysezelle überführt, in der die gelösten Titan-(IV)-salze wieder zu Titan-(III)-salzen reduziert werden. Man erhält so 1500 Teile einer salzsauren Lösung, die 9,16 % $TiCl_3$ enthält. Diese Lösung wird mit 48 Teilen 2,2'-Dichlorazoxybenzol versetzt und 3 Stunden bei 55 - 60°C gerührt. Nach dem Abfiltrieren und Waschen gewinnt man 49 Teile reines 3,3'-Dichlorbenzidindihydrochlorid.

Beispiel 8

In 1160 Teilen 32 %iger Salzsäure werden 190 Teile Titantetrachlorid gelöst. Die Lösung wird mit 150 Teilen Wasser verdünnt und in eine Elektrolysezelle überführt, wo auf bekannte Weise das $TiCl_4$ zu $TiCl_3$ reduziert wird. Anschließend wird die Lösung außerhalb der Elektrolysezelle mit 5 Teilen Dimethyl-$C_{12}-C_{14}$-alkylbenzylammoniumchlorid und 45,2 Teilen 2,2'-Dichlorazoxybenzol versetzt. Nach 3 Stunden bei 55 bis 60°C können 46,2 Teile reines 3,3'-Dichlorbenzidindihydrochlorid isoliert werden. Die Mutterlauge wird dann erneut in die Elektrolysezelle gebracht, wo wiederum das Ti-(IV) zu Ti-(III) reduziert wird und für einen erneuten Reaktionskreislauf zur Verfügung steht.

## Patentansprüche

1. Verfahren zur Herstellung von substituierten 4,4'-Diaminodiphenylen der allgemeinen Formel

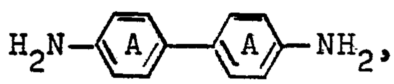

in der die Ringe A mindestens einen Substituenten tragen, aus Verbindungen der Formel II

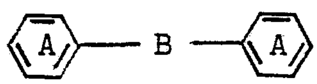 ,

in der
B einen Rest der Formeln

$$-N=N- \quad \text{oder} \quad -\overset{O}{\overset{\uparrow}{N}}=N$$

bedeutet, dadurch gekennzeichnet, daß man die Verbindungen der Formel II in stark saurem Medium mit Titantrichlorid umsetzt.

2. Verfahren gemäß Anspruch 1, dadurch gekennzeichnet, daß man eine salzsaure Lösung von Titantrichlorid verwendet.

3. Verfahren gemäß Anspruch 1 und 2, dadurch gekennzeichnet, daß man ein Tensid zusetzt.

4. Verfahren gemäß Anspruch 1 bis 3, dadurch gekennzeichnet, daß man das Reaktionsmedium elektrolytisch regeneriert und im Kreis führt.